Europäisches Patentamt

European Patent Office

Office européen des brevets

(11) Veröffentlichungsnummer: **0 224 655**

**A1**

(12) ## EUROPÄISCHE PATENTANMELDUNG

(21) Anmeldenummer: 86111727.3

(22) Anmeldetag: 25.08.86

(51) Int. Cl.4: **G01N 31/12** , G01N 33/22

(30) Priorität: 30.11.85 DE 3542377

(43) Veröffentlichungstag der Anmeldung:
10.06.87 Patentblatt 87/24

(84) Benannte Vertragsstaaten:
DE FR GB IT NL SE

(71) Anmelder: **Dr.Ing.h.c. F. Porsche Aktiengesellschaft Porschestrasse 42 D-7000 Stuttgart 40(DE)**

(72) Erfinder: **Höchsmann, Günther, Dipl.-Ing. Schillerstrasse 17 D-7126 Sersheim(DE)**

(54) **Verfahren zum Bestimmen der stöchiometrischen Luftmengen von Kraftstoffen.**

(57) Zur Bestimmung der stöchiometrischen Luftmengen von Kraftstoffen wird in einen Brenner ein gemessener Luftstrom und ein dosierter Kraftstoffstrom eingeleitet und verbrannt. Das Abgas des Brenners wird an einer Lambda-Sonde vorbeigeführt. Durch Dosieren des Kraftstoffstromes wird der Lambda-Wert auf 1 eingestellt und die stöchiometrische Luftmenge als Quotient aus Luftmassenstrom und Kraftstoffmassenstrom errechnet.

EP 0 224 655 A1

**Verfahren zum Bestimmen der stöchiometrischen Luftmengen von Kraftstoffen**

Die Erfindung betrifft ein Verfahren zum Bestimmen der stöchiometrischen Luftmengen von Kraftstoffen.

Die übliche Methode zur Bestimmung der stöchiometrischen Luftmengen, d.h. der Mindestluftmenge, die erforderlich ist, um eine bestimmte Kraftstoffmenge vollständig zu verbrennen, besteht darin, in einer Elementaranalyse den Kohlenstoff-, Wasserstoff-und Sauerstoffgehalt des Kraftstoffs zu bestimmen und hieraus die zur Verbrennung nötige Luftmenge zu berechnen. Dieses Verfahren ist sehr zeitaufwendig und nur mit teuren Analysegeräten durchführbar.

Es ist die Aufgabe der Erfindung, ein Verfahren zur Bestimmung der stöchiometrischen Luftmengen von Kraftstoffen zu entwickeln, das rasch zum Ergebnis führt und einen nur geringen apparativen Aufwand erfordert.

Eine Lösung dieser Aufgabe gelingt mit den Verfahrensschritten, wie sie im Kennzeichen des Anspruchs 1 aufgeführt sind. Wenn die einem Brenner zugeführte Luftmenge gemessen und die zugeführte Kraftstoffmenge so dosiert wird, daß das aus dem Brenner austretende Abgas einen Lambda-Wert von 1 hat, läßt sich die stöchiometrische Luftmenge $L_{st\ddot{o}ch}$ aus der einfachen Formel berechnen:

$$L_{st\ddot{o}ch} = \frac{Luftmassenstrom\ (kg/h)}{Kraftstoffmassenstrom\ (kg/h)}$$

Gegenüber dem herkömmlichen Meßverfahren hat dieses Verfahren zwei entscheidende Vorteile: es ist mit geringem apparativem Aufwand ausführbar, da keine teueren Analysegeräte erforderlich sind und das Meßergebnis liegt nach wenigen Minuten vor. Aus DE-PS 30 01 308 ist eine Vorrichtung bekannt, bei der einem Brenner, in den Kraftstoff und Luft eingeleitet wird, ein Luftmengenmesser und ein Abgastemperaturfühler zugeordnet sind. Dabei wird in Verbindung mit einem Mikroprozessor die einer optimalen Verbrennung entsprechende Luftmenge bei vorgegebener Menge eines verdampften Kraftstoffs eingeregelt. Da diese Regelvorrichtung ohne Lambda-Sonde arbeitet, ist es mit ihr nicht möglich, die stöchiometrische Luftmenge auf so einfache Weise labormäßig zu bestimmen, wie es die Erfindung vorsieht.

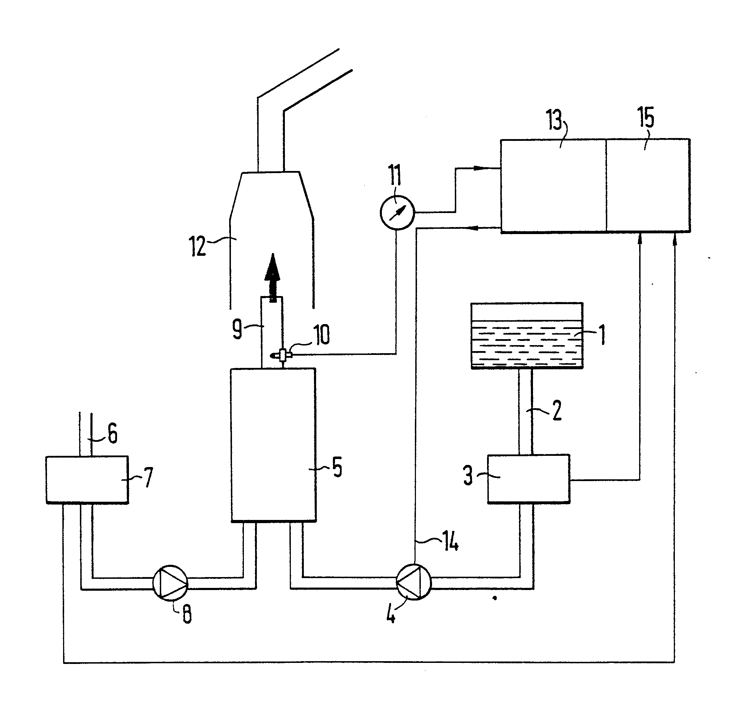

Eine Vorrichtung zur Ausführung des erfindungsgemäßen Verfahrens ist in der Zeichnung schematisch dargestellt und wird nachfolgend erläutert.

Aus einem Kraftstoffbehälter 1 fließt Kraftstoff über eine Kraftstoffzuleitung 2 in einen Kraftstoffmengenmesser 3 und weiter zu einer Dosierpumpe 4, die den Kraftstoff zu einem Brenner 5 fördert. Im Brenner 5 wird der Kraftstoff verdampft und unter Luftzufuhr verbrannt. Die Luft gelangt über eine Luftleitung 6 in einen Luftmengenmesser 7 und wird durch eine Luftpumpe 8 in den Brenner 5 gepumpt. Das aus dem Brenner 5 austretende Abgas wird an einer in einer Abgasleitung 9 eingesetzten Lambda-Sonde 10 vorbeigeführt, die ein Lambda-Signal auf ein elektrisches Anzeigegerät 11 gibt. Das aus der Abgasleitung 9 strömende Abgas wird in einer Absaugvorrichtung 12 aufgefangen.

Zur Bestimmung der stöchiometrischen Luftmenge wird an der Dosierpumpe 4 die dem Brenner 5 zugeführte Kraftstoffmenge so eingestellt, daß der am Anzeigegerät 11 ersichtliche Lambda-Wert genau = 1 ist. Am Luftmengenmesser 7 und am Kraftstoffmengenmesser 3 werden die Werte für den Luftmassenstrom sowie den Kraftstoffmassenstrom abgelesen. Der aus beiden Werten errechnete Quotient stellt die stöchiometrische Luftmenge dar.

Um die stöchiometrische Luftmenge vollautomatisch bestimmen zu können, ist ein Regelgerät 13 vorgesehen, das über das Anzeigegerät 11 an die Lambda-Sonde 10 angeschlossen ist und von ihr Meßsignale erhält. Durch eine Steuerleitung 14 ist das Regelgerät mit der Dosierpumpe 4 verbunden und stellt sie so ein, daß ein Lambdawert von 1 eingeregelt wird.

Ein Rechengerät 15, das mit dem Regelgerät 13 eine Baueinheit bildet, ist durch parallele Leitungen mit dem Kraftstoffmengenmesser 3 und dem Luftmengenmesser 7 verbunden. Es bildet den Quotienten dieser beiden Meßsignale und zeigt die entsprechende stöchiometrische Luftmenge an.

**Ansprüche**

1. Verfahren zum Bestimmen der stöchiometrischen Luftmengen von Kraftstoffen, gekennzeichnet durch die Verfahrensschritte

a) einem Brenner wird eine dosierbare Kraftstoffmenge zugeführt, deren Massenstrom (kg/h) in der Kraftstoffzuleitung (2) zum Brenner (5) gemessen wird,

b) der Kraftstoff wird in dem Brenner (5) in Luft verbrannt, die in den Brenner eingeblasen wird und deren Massenstrom (kg/h) in einer Luftleitung (6) vor Eintritt in den Brenner (5) gemessen wird,

c) das aus dem Brenner (5) austretende Abgas wird an einer Lambda-Sonde (10) vorbeigeführt, die ein Meßsignal auf ein Anzeigegerät (11) gibt,

d) die zugeführte Kraftstoffmenge wird so dosiert, daß der angezeigte Lambda-Wert = 1 ist,

e) die stöchiometrische Luftmenge $L_{st}$ öch des Kraftstoffs wird aus dem Quotienten des gemessenen Luftmassenstroms (kg/h) und des Kraftstoffmassenstroms (kg/h) berechnet.

2. Verfahren nach Anspruch 1, dadurch gekennzeichnet, daß die zum Erreichen eines Lambda-Werts von 1 erforderliche Kraftstoffmenge mit einer in der Kraftstoffzuleitung (2) zum Brenner (5) eingebauten Dosierpumpe (4) eingestellt wird.

**Europäisches Patentamt**

**EUROPÄISCHER RECHERCHENBERICHT**

Nummer der Anmeldung

EP 86 11 1727

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | FR-A-2 564 591 (INSTITUT DE RECHERCHES DE LA SIDERURGIE FRANCAISE) * ganzes Dokument * | 1 | G 01 N 31/12 G 01 N 33/22 |
| A | EP-A-0 086 581 (HONEYWELL INC.) * Seite 1, Zeilen 4-22; Seite 2, Zeilen 13-32; Seite 3, Zeilen 3-8; Ansprüche * | 1,2 | |
| A | EP-A-0 031 145 (HONEYWELL INC.) * Seite 7, Zeile 16 - Seite 8, Zeile 25; Seite 13, Zeile 7 - Seite 14, Zeile 23; Seite 29, Zeile 13 - Seite 30, Zeile 13 * | 1 | |
| A | EP-A-0 107 341 (HONEYWELL INC.) * ganzes Dokument * | 1 | **RECHERCHIERTE SACHGEBIETE (Int. Cl.4)** |
| A | US-A-3 738 808 (G.P. CUNNINGHAM et al.) * Spalte 3, Zeile 21 - Spalte 4, Zeile 75 * | 1,2 | G 01 N 31/00 G 01 N 33/00 G 01 N 25/00 |
| A | EP-A-0 098 716 (THE BABCOCK & WILCOX CO.) | | |

Der vorliegende Recherchenbericht wurde für alle Patentansprüche erstellt.

| Recherchenort | Abschlußdatum der Recherche | Prüfer |
|---|---|---|
| BERLIN | 08-01-1987 | VINSOME R M |